# EUROPEAN PATENT SPECIFICATION

(11) **EP 0 732 959 B1**
(45) Date of publication and mention of the grant of the patent: **09.09.1998**
(21) Application number: 95902105.6
(22) Date of filing: 01.12.1994
(51) Int. Cl.: A61M 37/00, A61M 5/14, A61B 5/14

(54) **DEVICE FOR USE IN TRANSDERMAL PERFUSION PROCEDURES**
VORRICHTUNG ZUM GEBRAUCH BEI TRANSDERMISCHEN INFUSIONEN
DISPOSITIF DESTINE AUX PROCEDES DE PERFUSION TRANSDERMIQUE

(30) Priority: 07.12.1993 GB 9325085; 10.05.1994 GB 9409285
(43) Date of publication of application: 25.09.1996
(60) Divisional application: 98100950.9
(73) Proprietor: SVEDMAN, Pal, 1255 Veyrier (CH)
(72) Inventor: SVEDMAN, Pal, 1255 Veyrier (CH)
(74) Representative: Baldock, Sharon Claire
(86) International application number: EP9403986
(87) International publication number: WO9515783

(56) References cited:
- EP-A- 0 555 554
- EP-A- 0 625 360
- WO-A-92/11879
- CH-A- 662 740
- DE-A- 2 018 468
- DE-A- 3 019 589
- DE-C- 57 457
- GB-A- 992 424
- US-A- 4 404 924
- US-A- 5 336 213

## Description

This invention relates to a device for use in transdermal perfusion procedures and in particular but not exclusively to a device for use in the transdermal absorption of substances through the skin of human or animal bodies.

Transdermal procedures have hitherto been limited in their usefulness by the relatively impermeable nature of the outer layer or the skin (epidermis or epithelium).

It is known from WO 92/11879 to provide apparatus which allows a suction blister to be formed on a patient's skin by the application of suction over a prolonged period, means being provided for removing a roof of the blister to expose an area of dermis at a treatment site. The absorption of substances through the dermis into the body or sampling of plasma exuded from the body via the dermis is then facilitated in a painless and minimally invasive manner during a further period prior to growth of a new covering of epidermis.

DE-C-57457 discloses a syringe for creating suction consisting of a cylinder with a piston which is movable by means of an actuating handle which is integral with a locking arm which can be used to lock the piston in the retracted position.

US 4,404,924 describes a medical suction device including a flag to indicate the pressure being developed by the suction device.

DE 3019589 discloses a device for transdermal perfusion which includes a syringe for delivering liquid to the perfusion site.

CH 662740 discloses a device for transdermal perfusion which includes means to control the temperature of the treatment site.

EP 555554 discloses a blood sampling device for attachment to the skin which includes a sampling chamber to which suction can be applied.

The process of removing a portion of epidermis, also referred to as an epithelial layer, from the underlying intact dermis is referred to below as a de-epithelialising method and the resulting area of skin from which the epidermis has been removed will be referred to as a de-epithelialised erosion or lesion. Such an erosion formed by splitting the epidermis from the dermis by suctioning followed by removal or disruption of the detached epidermis allows a unique form of access to the body. The suctioning process causes a spit to occur reproducibly through the lamina lucida part of the basal membrane between the epidermis and the dermis. Although the thickness of the epidermis varies over different parts of the body, the epidermis over most areas is so delicate and thin as to be transparent. Irrespective of the thickness of the epidermis however, the split occurs at the basal lamina level. The dermis may vary in thickness also but this variation similarly does not affect the level at which the split occurs.

The portion of epidermis which is removed in such a method is devoid of vessels and nerves and is so delicate and thin as to be transparent. The underlying connective tissue of the lamina densa with scattered islands of epithelial adnexae is left behind. The lamina densa is a robust fibrous layer which is open to molecular passage but structurally remains a safeguard to the underlying dermis which remains structurally completely intact.

The blood flow in the exposed dermis is strongly increased for several days following formation of such an erosion allowing increased macro molecular passage, the lymphatic system of the dermis remaining intact and the lymph vessels allowing free passage of even the largest plasma proteins.

The split is formed after exposing the skin surface to a pressure of 26.664 kP (200 millimetres of mercury) below atmospheric pressure for a period of two to two and a half hours. This period may be reduced to less than one hour by local heating.

During the latter part of the suctioning period, the split fills with clear liquid emanating from the underlying microvessels, finally forming a fluid filled generally semi-spherical blister covered externally by the detached epithelial layer.

This detached epithelial layer must be broken or removed in order to provide access for drug absorption or sampling. The exposed dermis will readily absorb fluid substances brought into contact with the dermis at the erosion, the active ingredients of such substances typically being transdermally absorbed by diffusion through the dermis in response to a concentration gradient of the ingredient existing across the thickness of the dermis, and the substances being rapidly dispersed through the body via the blood vessels of the dermis. A clear fluid exudate continues to form at the erosion by plasma passing outwardly through the dermis. This exudate typically can be analysed to monitor levels of natural or artificial substances in the body. A variety of transdermal perfusion procedures involving the passage of substances through the exposed dermis are thereby envisaged. The term perfusion in the present context is used to encompass such diffusion either into or from the body in either a natural or enhanced manner.

The present invention relates to an improved apparatus which can be safely and reliably operated in a simple manner such that in many instances patients themselves may be left to carry out the stages of operation required to complete the procedure.

According to the present invention there is disclosed apparatus for use in transdermal perfusion procedures comprising a housing, securing means operable to secure a contact surface of the housing in sealing contact with an area of skin in use, an aperture defined in the contact surface, a suction chamber communicating with the aperture and suction means operable to apply suction to the suction chamber, characterised in that the aperture communicates with an access port defined by the housing, a suction cup is located in the access port and has a lip portion extending peripherally of the aperture and the suction cup defining an outlet port, wherein the suction means is operable to apply suction to the suction chamber in the outlet port and the apparatus further comprises cutting means operable to sever from the lip portion a removable portion of the suction cup defining the outlet port.

An advantage of such apparatus is that a suction blister formed within the suction chamber can be safely cut at the same time or the removable portion is severed in a controlled manner by operation of the cutting means and such that a roof of the blister may be disposed of by subsequent removal of the removable portion of the suction cup. The roof of the blister is constituted by an area of epidermis of the skin detached by the formation of the suction blister from the underlying dermis so that following removal of the roof of the blister a small skin erosion is formed providing direct access to the intact dermis for a transdermal perfusion procedure which may involve placing a fluid in contact with the dermis for absorption into the body or may alternatively involve the collection and sampling of exudate eminating from the erosion.

Preferably the apparatus further comprises an actuator mounted on the housing and the cutting means comprises a blade which is movable to sever the removable portion in response to movement of the actuator relative to the housing.

The blade may thereby be safely moved in a controlled manner.

Preferably the suction means defines an expansion chamber communicating with the outlet port via a tube, the suction means being operable to expand the volume of the expansion chamber from an initial volume to an expanded volume and further comprising locking means operable to maintain the expansion chamber in its expanded volume.

Suction within the suction chamber may thereby be applied over a prolonged period by operation of the locking means to maintain the expansion chamber in its expanded condition.

Advantageously the suction means comprises a syringe.

Advantageously the apparatus further comprises an indicator responsive to displacement of air along the tube and operable to provide an indication of volumetric displacement of air from the suction chamber in response to the formation of a suction blister within the suction chamber.

An advantage of such an indicator is to allow the formation of the suction blister to be remotely monitored. This is important since the time taken for the formation of a suction blister of a given size will vary from patient to patient and will vary according to other prevailing operating conditions such as temperature. Visual inspection of the suction blister will not generally be a convenient option.

Conveniently the indicator comprises a slug of liquid contained within the tube and indicating means for indicating the extent of linear displacement of the slug of liquid through the tube.

Conveniently the indicator comprises a clamp securable at an adjustable position along the tube and wherein the indicating means is supported by the clamp.

Preferably the suction means comprises a connector which is releasably engagable with the outlet port whereby disengagement of the connector from the outlet port admits ambient air to the suction chamber.

An advantage of such a connector is to allow the suction means to be dissociated from the housing and discarded by the patient after the formation of the suction blister.

Preferably the apparatus comprises an arming device operable to prevent actuation of the cutting means until the connector has been disconnected from the outlet port.

An advantage of such an arming device is to ensure that the application of suction within the suction chamber is discontinued prior to actuation of the cutting means.

This is a safety feature which avoids the possibility of damaging the underlying dermis. Without this safety feature, there would be the possibility that suction within the suction chamber could deform the dermis into a bulbous projection extending into the suction chamber to an extent such that it could be cut by the blade during the blister cutting operation.

Conveniently the arming device comprises an arming pin insertable into the housing to a location in which it prevents relative movement of the actuator and housing, the arming device further comprising a handle connected to both the connector and the arming pin.

Preferably the housing comprises a base defining the contact surface and a rotatable portion in which the access port is defined at an eccentric location relative to the rotation axis of the rotatable portion.

The rotatable portion may then comprise a reservoir having an outlet located eccentrically relative to the rotational axis such that, after the removable portion has been removed, the outlet is locatable by rotation of the rotatable portion in registration with the aperture defined in the base.

Advantageously a continuous seal extends peripherally of the outlet and is operable between the rotatable portion and the base.

The seal thereby allows the contents of the reservoir to be sealed against loss of contents and ingress of air during an initial period in which the outlet is overlaid by a continuous surface of the base. Subsequently the seal provides sealing action peripherally of the aperture defined in the base when the outlet is in registration with the aperture, loss of fluid thereby being prevented together with the prevention of ingress of contaminants.

Advantageously the apparatus further comprises a second continuous seal peripheral to a surface portion of the rotatable portion at a location which is eccentric relative to the rotational axis such that the surface portion is movable by rotation of the rotatable portion into registration with the aperture.

The surface portion of the rotatable portion may thereby be used to form a closure to the suction chamber after use of the apparatus to deliver the contents of the reservoir to the dermis, in this final position the second continuous seal being operable to form an airtight seal between the surface portion and the base against the ingress of contaminants.

Conveniently the continuous seal and the second continuous seal are integrally formed.

Advantageously the suction cup comprises a cylindrical portion intermediate the lip and the outlet port and having a cylindrical axis substantially orthogonal to the contact surface and the cutting means is operable to sever the cylindrical portion at a predetermined location spaced from the lip portion.

Preferably the suction cup comprises an internal surface which is adhesively coated.

A suction blister will therefore tend to adhere to the internal surfaces of the suction cup thereby avoiding the collapse of the blister before cutting in the event of the blister becoming accidentally ruptured.

The rotatable portion may define a sampling port at an eccentric location relative to the rotation axis of the rotatable portion, the apparatus further comprising a sampling piston reciprocatable in the sampling port to vary the volume of a sampling chamber defined therein whereby suction may be created in the sampling chamber, the sampling port being locatable, after the removable portion has been removed, by rotation of the rotatable portion in registration with the aperture defined in the base such that the aperture communicates with the sampling chamber.

Exudate emanating from the exposed dermis may thereby be sampled via the sampling port without removal of the housing from the skin to which it is attached. On completion of sampling, the rotatable portion may then be rotated into a position in which the delivery of a drug commences or recommences, or alternatively may be rotated into a position in which the erosion is occluded.

The ability of the sampling piston to create suction in the sampling chamber may be utilised to enhance the rate at which exudate is collected from the erosion by increasing the rate of production of exudate in response to a pressure differential applied across the dermis.

Conveniently the sampling piston defines a bore to receive an outflow of fluid from the sampling chamber, the apparatus further comprising an openable closure operable to close the bore to maintain suction in the sampling chamber.

Access to the exudate fluid in the sampling chamber may thereby be gained by opening the closure as required.

Conveniently the apparatus further comprises a sampling device connected to the sampling piston and defining a sampling channel communicating in use with the bore to receive a sample of fluid from the sampling chamber.

Advantageously the sampling piston is reciprocatable in the sampling port by means of co-operable screw threaded formations of the sampling piston and the rotatable portion.

Preferably, the suction means comprises a syringe which comprises a cylinder within which a piston is movable to define an expansion chamber of variable volume, the piston comprising an actuating handle projecting from the cylinder and a locking arm formed integrally therewith, the locking arm being resiliently biased for movement into a locking position in which a free end of the locking arm engages a co-operating looking formation of the cylinder when the piston is in a retracted position to create and maintain suction in the expansion chamber.

Such a syringe nay be conveniently locked for a prolonged period with the piston in the retracted position to thereby maintain suction during the formation of a suction blister.

Advantageously the apparatus comprises an actuating mechanism operable to facilitate rotational movement of the rotatable portion relative to the base in response to movement of an actuating member of the mechanism.

The mechanism may comprise a geared pinion mounted on the base for rotation by movement of the actuator and a circumferential rack mounted on the rotatable portion and engaged by the pinion.

Preferred embodiments of the present invention will now be described by way of example only and with reference to the accompanying drawings of which
Figure 1 is a schematic sectional elevation of a device showing the formation of a suction blister within a suction cup;
Figure 2 is a schematic sectional elevation of the device of Figure 1 after the suction cup and blister have been severed;
Figure 3 is a sectioned elevation of the device of preceding figures during a drug delivery phase of operation;
Figure 4 is a sectional elevation of the device of preceding figures shown after completion of the drug delivery phase.
Figure 5 is a perspective view of the suction cup and arming device of the device of preceding figures;
Figure 6 is a sectional elevation of the device of preceding figures with the suction cup removed;
Figure 7 is a plan view of a base of the device of preceding figures;
Figure 8 is a sectional elevation of the base of Figure 7;
Figure 9 is a plan view of a rotatable portion of the device of preceding figures;
Figure 10 is a sectional elevation of the rotatable portion of Figure 9;
Figure 11 is a plan view of a cover plate for the rotatable portion of Figures 9 and 10;
Figure 12 is a sectional elevation of the cover plate of Figure 11;
Figure 13 is a plan view of an actuating ring of the device of Figures 1 to 5;
Figure 14 is a sectional elevation of the actuating ring of Figure 13;
Figure 15 is a plan view of the device of preceding figures with the suction cup omitted and showing the device in a rest position prior to removal of the arming device;
Figure 16 is a plan view of the arrangement of Figure 15 with the arming device removed;
Figure 17 is a plan view of the arrangement of Figure 15 after rotation of the actuating ring to a position in which the blade is moved through the access port in which the suction cup is locatable;
Figure 18 is a plan view of the arrangement of Figure 15 showing a further position in which the rotatable portion has been rotated to bring an outlet port of the reservoir into registration with the access port;
Figure 19 is a plan view of the arrangement of Figure 15 following further movement of the rotatable portion to a position in which the access port is sealed;
Figure 21 is a plan view of an alternative device having a rotatable key for rotating the rotatable portion;
Figure 27 is a partly section elevation of a syringe for use in the device of Figure 5;
Figure 28 is a partly sectioned plan view of the syringe of Figure 27;
Figure 29 is a partly sectioned plan view of the device of the syringe of Figures 27 and 28 in a retracted configuration;
Figure 66 is an underneath plan view of a rotatable portion of an alternative device, the rotatable portion being cooperable with a base of the type shown in Figure 7 and an actuating ring of the type shown in Figure 13;
Figure 67 is a plan view of the rotatable portion of Figure 66 with the cover plate removed;
Figure 68 is a schematic plan view of the device of Figures 66 and 67;
Figure 69 is a sectional elevation of the device shown in Figure 68 with the sampling port not in use;
Figure 70 is a plan view of the device of Figures 66 to 69 showing the rotatable portion moved into a position where the sampling port is in use;
Figure 71 is a sectional elevation of the device shown in Figure 70 showing the sampling port in use;
Figure 72 is a sectional elevation of the device of Figure 71 after retraction of the sampling piston to a fully retracted position;
Figure 73 is a sectional elevation of the device of Figure 72 after advancing the sampling position to an intermediate position;
Figure 74 is a sectional elevation of the device of Figures 66 to 73 after fitting of a cuvette to the sampling piston;
Figure 75 is a side elevation of the cuvette of Figure 74;
Figure 76 is a sectional elevation showing the device of Figure 74 with the sampling piston fully advanced;
Figure 77 is a sectional elevation of the cuvette of Figures 74 to 76 after removal from the device and insertion into a photometer;
Figure 78 is a sectional elevation of the device of Figures 66 to 77 in which a capillary active plug is fitted to the sampling piston;

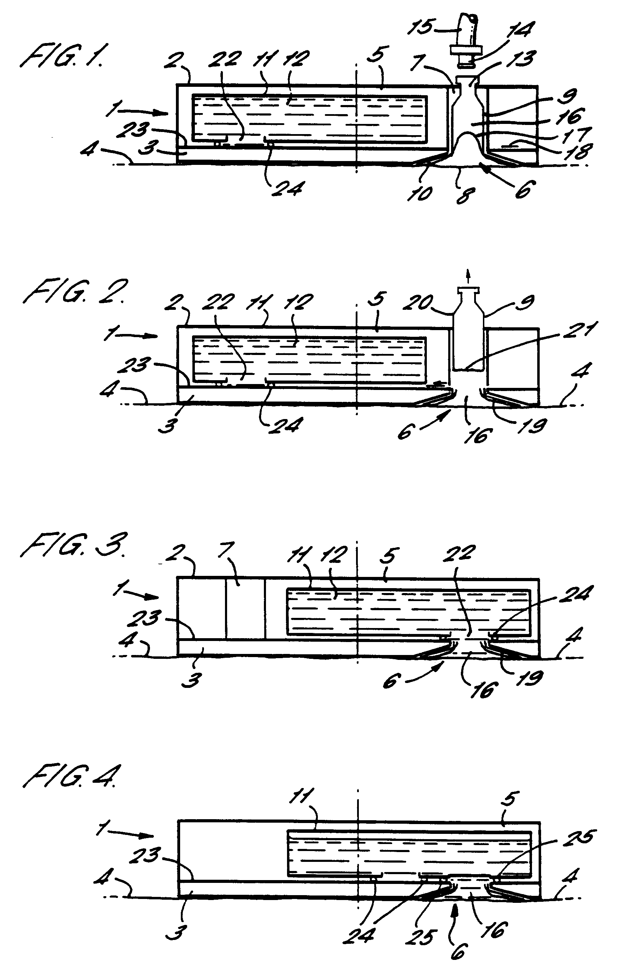

Figure 1 shows a device 1 for use in the transdermal delivery of a liquid drug in contact with a de-epithelialised area of skin and for initially de-epithelialising the skin by formation and subsequent disruption of a suction blister.

The device 1 comprises a housing 2 consisting of a base 3 secured in contact with a patient's skin 4 and having a rotatable portion 5. The base 3 is disc shaped and the rotatable portion 5 generally cylindrical and coupled to the base so as to be rotatable relative to the base about its cylindrical axis in continuous sliding contact with the base.

A circular aperture 6 is defined in the base 3 at a location eccentric relative to the cylindrical axis of the rotatable portion 5. In the rest position of the device shown in Figure 1, a cylindrical access port 7 defined in the rotatable portion 5 is aligned in communication with the aperture 6 such that a circular area of skin 8 is accessible through the device 1.

A suction cup 9 is located in the access port 7 and has a flared lip 10 of greater diameter than the port 7 such that the suction cup is captively retained. The base 3 is recessed peripherally of the aperture 6 to accommodate the lip 10. The internal surfaces of the cup 9 are coated with acrylic adhesive so that, once a suction blister is formed in the cup, the surface of the blister will adhere to the cup, thereby maintaining the blister in an elevated position. This will tend to prevent collapse of the blister in the event of accidental rupture.

The rotatable portion 5 also accommodates a reservoir 11 containing a liquid drug, the reservoir 11 being isolated from the access port 7 in the position shown in Figure 1.

The suction cup 9 has a female connector 13 which is engagable with a male connector 14 of a suction tube 15 via which suction can be applied in use to a suction chamber 16 defined within the suction cup 9. In Figure 1, the device is shown in its rest position following the application of suction within the chamber 16 for a period sufficient to result in the formation of a suction blister 17, following which the male and female connectors 13 and 14 have been disconnected from one another to admit air at ambient pressure to the chamber 16.

The device 1 also includes a blade 18 which extends radially with respect to the cylindrical axis of the rotatable portion 5 and which is movable by rotation about the cylindrical axis in a plane defined by the interface between the base 3 and the rotatable portion 5.

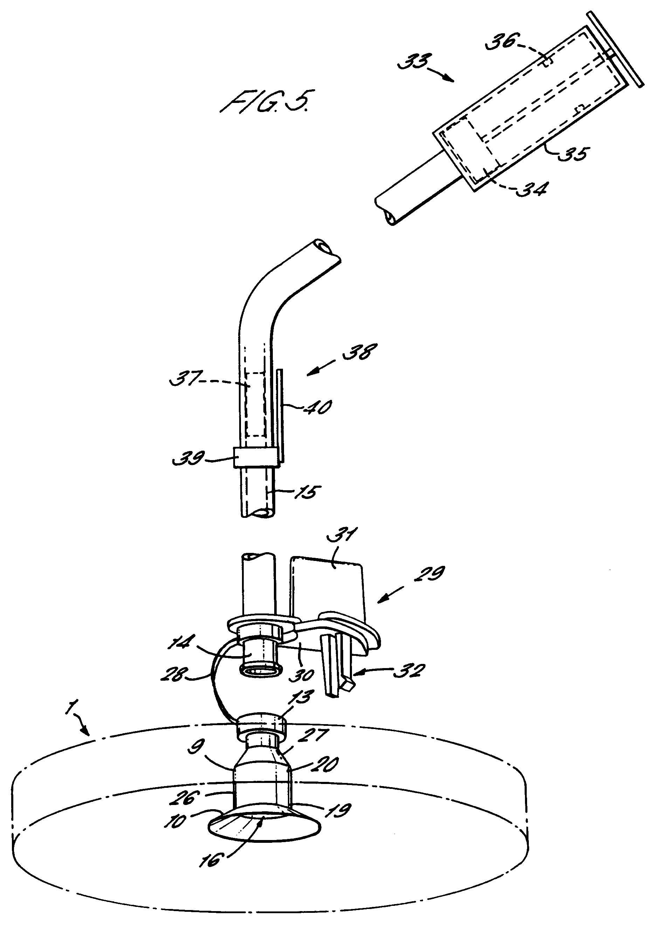

In Figure 2 the operation of the blade 18 is illustrated in that it has been moved arcuately so as to cut through the suction cup 9 at a location which is intermediate the roof of the suction blister 17 and the aperture 6. By this cutting action, the suction cup 9 is severed into a captive portion 19 extending through the base 3 and a removable portion 20 extending through the rotatable portion 5 and to which the roof 21 of suction blister 17 remains adhered.

By withdrawing the removable portion 20 from the access port 7, the roof 21 may thereby be disposed of. Since the removable portion 20 is severed at the interface between the base 3 and the rotatable portion 5, the severing action of the blade 18 thereby allows the rotatable portion 5 to be subsequently movable by rotation relative to the base 3 whereas previously the presence of the suction cup extending through the aperture 6 and access port 7 prevented such relative rotational movement about the cylindrical axis.

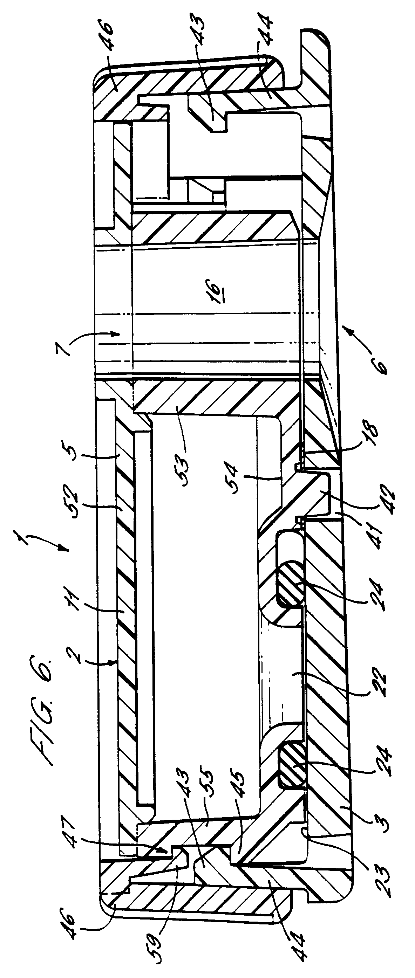

The reservoir 11 has an outlet port 22 which in the rest position of the rotatable portion 5 as shown in Figures 1 and 2 is closed by an upper surface 23 of the base 3, a continuous O-ring seal 24 being interposed between the surface 23 and the rotatable portion 5 to prevent peripheral leakage from the outlet port 22. Following the severing of the suction cup 9, the rotatable portion 5 is rotated into a second position shown in Figure 3 in which the outlet port 22 is brought into registration with the aperture 6, the location of the outlet port 22 being spaced radially from the cylindrical axis of the rotatable portion 5 about which it is rotated.

The O-ring seal 24 is maintained in a fixed position relative to the rotatable portion 5 so that in this second position it forms a peripheral barrier between the rotatable portion 5 and the upper surface 23 of the base 3. The drug 12 within the reservoir 11 then enters the chamber 16 and comes into contact with the area of skin 8 which has been de-epithelialised following removal of the suction blister 17. The device 1 is retained in this second position during a drug delivery phase of operation in which the drug is absorbed into the patient.

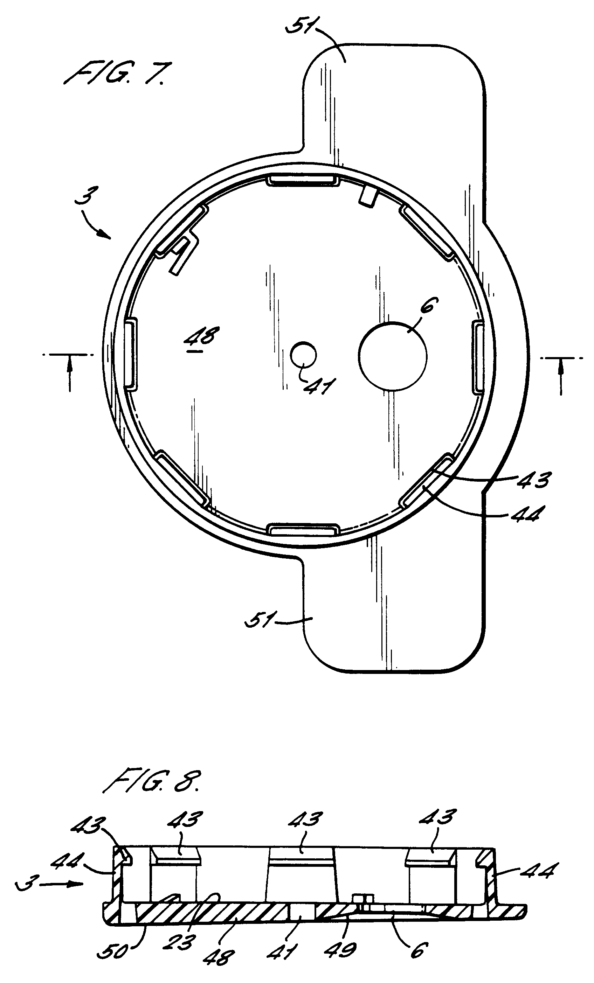

On completion of this phase, the rotatable portion 5 is again rotated and moved into a third position shown in Figure 4. In this third position, the outlet port 22 of the reservoir 11 is again closed by the upper surface 23, assisted by the sealing action of the O-ring seal 24. A second O-ring seal 25 is brought into peripheral sealing engagement between the upper surface 23 and the rotatable portion 5 at a location peripheral to the aperture 6 thereby providing an air tight seal to the chamber 16.

The rotatable portion 5 may subsequently be returned to the second position should further drug delivery be required or the device 1 may be removed from the patient on completion of the procedure.

The above Figures 1 to 4 are schematic in nature and serve to illustrate the function of the above mentioned elements. The detailed construction of these elements will now be described in the context of a specific embodiment of the invention.

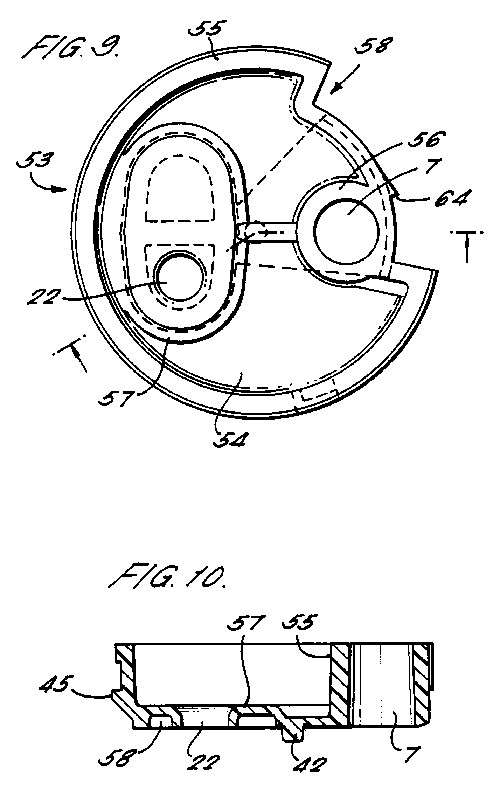

In Figure 5 the suction cup 9 is shown to comprise a cylindrical portion 26 defining the chamber 16 and which in use is severed radially by action of the blade 18 into captive and removable portions 19 and 20. The internal surfaces of the suction cup 9 are coated with an acrylic adhesive incorporated with random oriented polyester fibres.

The removable portion 20 terminates in the female connector 13 to which it is connected by a frustoconical tapered portion 27. The male connector 14 is connected by a flexible web 28 to the female connector 13 such that when disconnected from one another they are retained in loose association. An arming device 29 is connected to the male connector 14 and consists of a plate 30 through which the suction tube 15 passes, the plate being formed integrally with an outwardly projecting handle 31 and an oppositely projecting bifurcated arming pin 32. The arming device 29 is shaped such that it must be inserted within the rotatable portion 5 in order for the male and female connectors 14 and 13 to be engaged, the presence of the inserted arming pin 32 being arranged to prevent movement of the blade 18 from its initial position as shown in Figure 1. This arrangement thereby ensures that the blade 18 cannot be moved until the arming device 29 has been disengaged from the rotatable portion 5 and this disengagement also necessitates disengagement of the male and female connectors 14 and 13 so that suction can no longer be maintained in the chamber 16. This is a safety feature of the device 1 which is intended to avoid cutting the suction blister 17 while there is any suction within the chamber 16 which could displace the underlying dermis into a position in which it extends into the chamber sufficiently to be damaged by the blade.

The suction tube 15 is connected to a syringe 33 comprising a piston 34 slidable within a cylinder 35 and spring biassed into the position shown in Figure 5 in which the syringe volume is a minimum. The syringe is provided with a locking mechanism 36 enabling the piston 34 to be held in a withdrawn position corresponding to maximum syringe volume so that suction can be applied to the chamber 16 by engaging the male and female connectors 14 and 13 and then withdrawing the piston and locking the piston in place by means of the locking mechanism.

The suction tube 15 is formed of a transparent and flexible plastics material and contains a slug of liquid 37 forming part of an indicator 38.

The indicator 38 comprises a clamping ring 39 which is a tight fit on the external surface of the suction tube 15 but can be adjusted in position along the length of the tube so as to bring into registration with the slug of liquid 37 a linear scale 40. When suction is initially applied to the chamber 16 by action of the syringe 33, the position of the slug of liquid 37 will move due to displacement of air along the tube 15 to a new position and the operator using the device 1 at this time adjusts the position of the clamping ring 39 such that the end of the slug of liquid 37 is aligned with a zero marking on the scale 40. Suction is maintained with the chamber 16 during a blister forming period in which the suction blister 17 will progressively form and grow in size until it extends into the cylindrical portion 26. In doing so the blister 17 will displace air within the tube 15 and consequently the slug of liquid 37 will be linearly displaced relative to the scale 40. The scale 40 is calibrated such that the operator is able to determine the extent of displacement of the slug of liquid 37 corresponding to the blister 17 being fully formed to a predetermined level at which a predetermined volumetric displacement within the chamber 16 is achieved.

By visual inspection of the indicator 38 it is therefore possible for the operator to determine when the blister forming phase of operation is completed.

At this stage the operator will grip the handle 31 and pull the arming device 29 so as to withdraw the arming pin 32 and at the same time to disconnect the male connector 14 from the female connecter 17. Suction within the chamber 16 will then be lost.

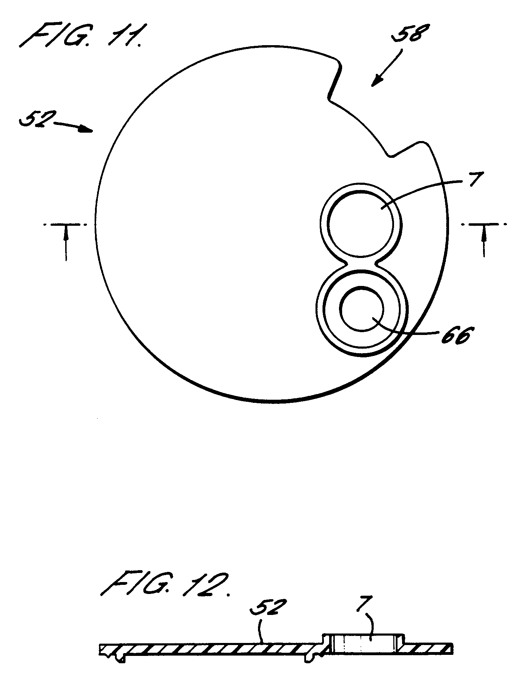

Figure 6 shows the device 1 in greater detail with the suction cup omitted for clarity. The base 3 has a central socket 41 in which an axially projecting pin formed integrally with the rotatable portion 5 is journaled thereby enabling the rotatable portion to be rotatable about its cylindrical axis relative to the base 3. The rotatable portion 5 is retained in axial abuttment with the base 3 by retaining flanges 43 which project radially inwardly from circumferentially spaced axial projections 44 formed integrally with the base 3.

The rotatable portion 5 is provided with a part annular rim 45 which projects radially outwardly so as to engage the projection 44.

An actuating ring 46 is mounted on the rotatable portion 5 and is captively retained by cooperating flange formations 44. The actuating ring 46 is capable of limited angular displacement relative to the rotatable portion 5 for the purpose of moving the blade 18 relative to the rotatable portion 5 and base 3 when the latter are locked together by the presence of the suction cup.

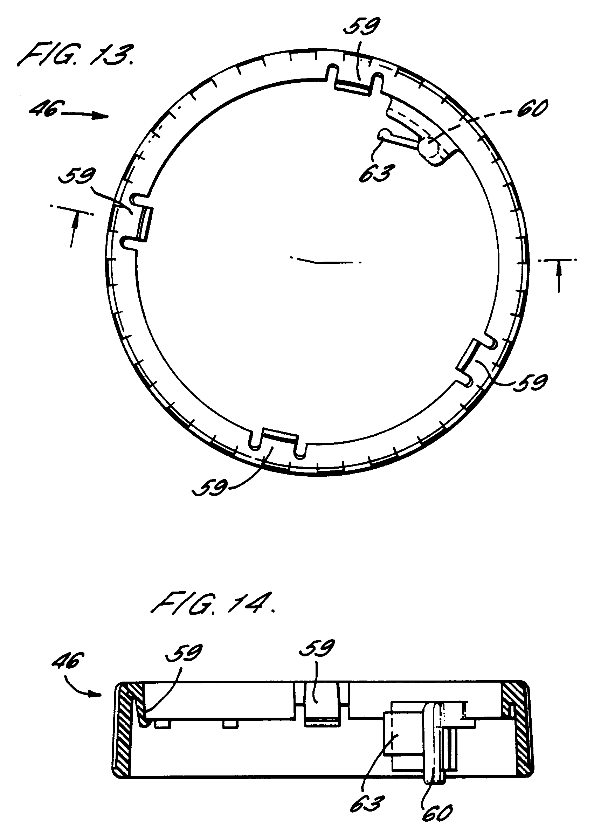

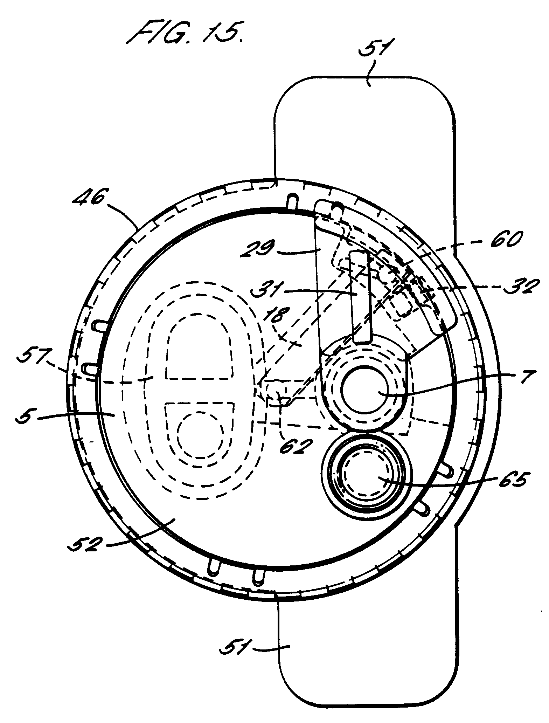

The shape of the base 3 is shown more clearly in Figures 7 and 8. The base 3 comprises a disc 48 in which the socket 41 is centrally formed and the aperture 6 is eccentrically located. A tapered seat 49 surrounds the aperture 6 and is shaped to conform to the lip 10 of the suction cup so that when the suction cup is inserted in the aperture 6 the lip 10 remains flush with the underside 50 of the disc 48. The disc 48 is provided with laterally projecting wings 51 to assist in stably securing the disc to the patient's skin. The underside 50 comprises an adhesive layer enabling the disc 48 to be secured to the skin in a secure and air tight manner so that in use with the suction cup evacuated the patient's skin forms a sealed closure to the chamber 16.

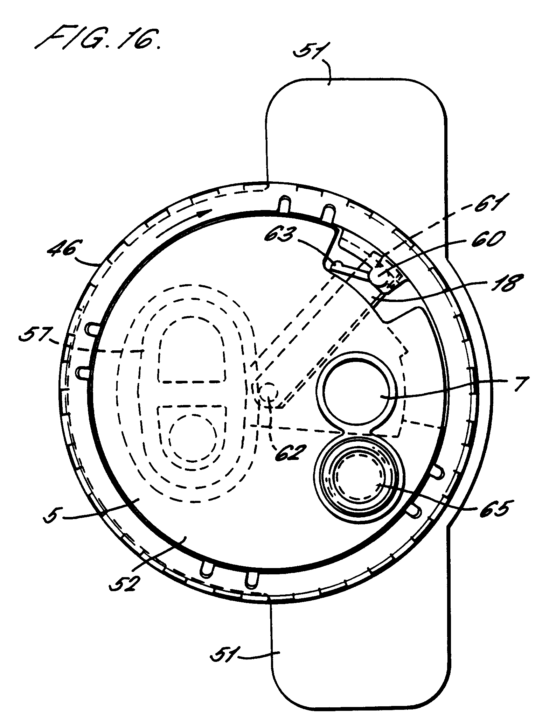

Figure 9 shows the rotatable portion 5 in plan view omitting a top cover plate 52 shown in Figure 6 which in the completed device is heat welded to a main body 53 of the rotatable portion 5 so as to close the reservoir 11. The main body 53 consists of a planar bottom 54 with an upstanding peripheral wall 55 which is part circular to define the rim 45 and includes a cylindrical wall portion 56 defining the access port 7.

The bottom 54 includes a raised figure of eight profile 57 which accommodates a recess 58 on the underside of the bottom 54 which receives integrally formed seals 24 and 25.

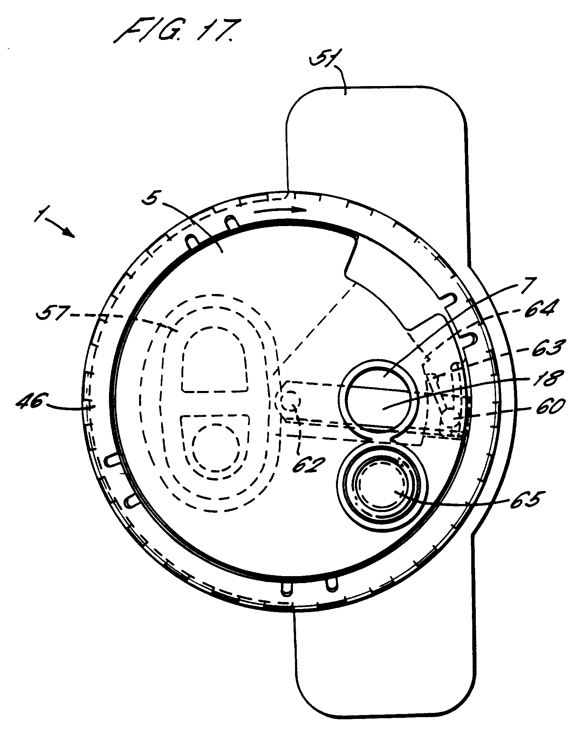

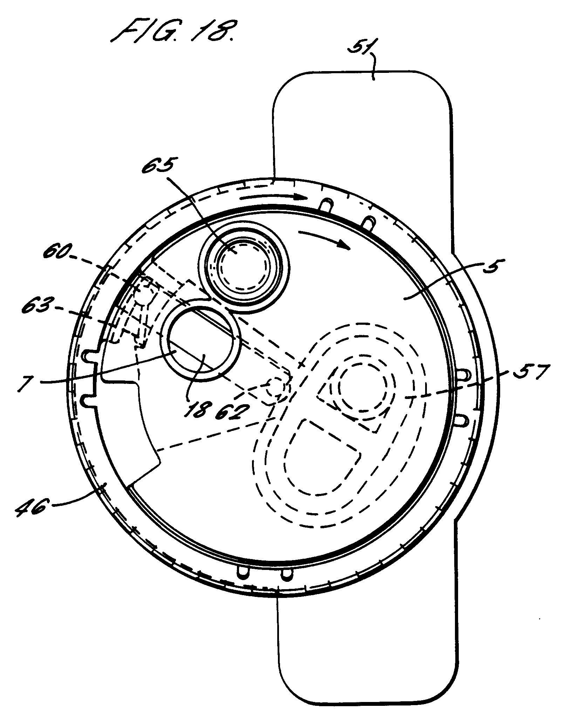

Figure 11 shows the shape of the cover plate 52 which is that of a disc with a cut out 58 provided to allow insertion of the arming pin 32.

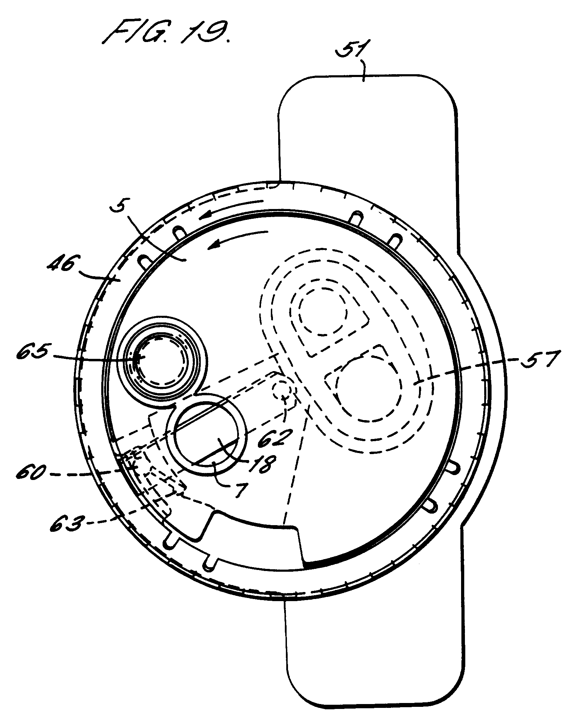

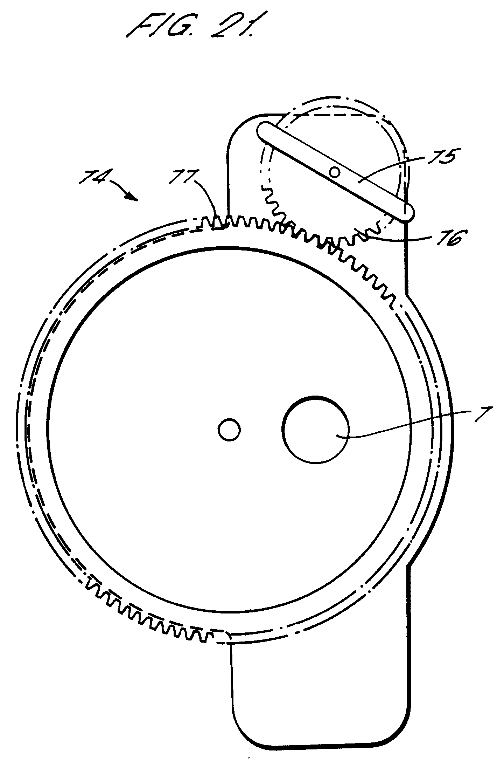

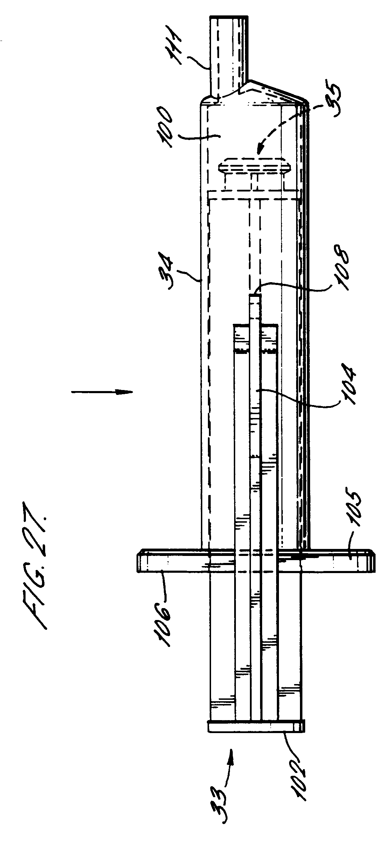

Figures 13 and 14 show the actuating ring 46 which has four circumferentially spaced snap fit retaining flanges 59 forming part of the flange formations 47 and thereby securing the actuating ring to the rotatable portion 5.

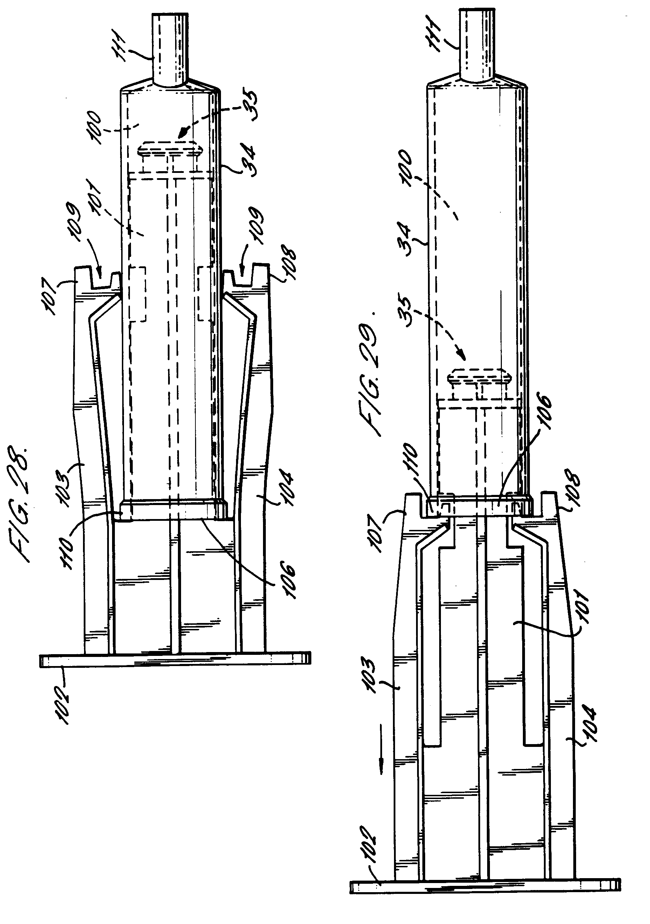

As shown in Figure 14 the actuating ring 46 includes a drive pin 60 which locates in a locating hole 61 at the outer radial extremity of the blade 18 as shown in Figure 15. Rotation of the actuating ring relative to the rotatable portion 5 will thereby cause the blade 18 to rotate about an inner radial pivot 63 which is coaxial with the cylindrical axis of the rotatable portion 5.

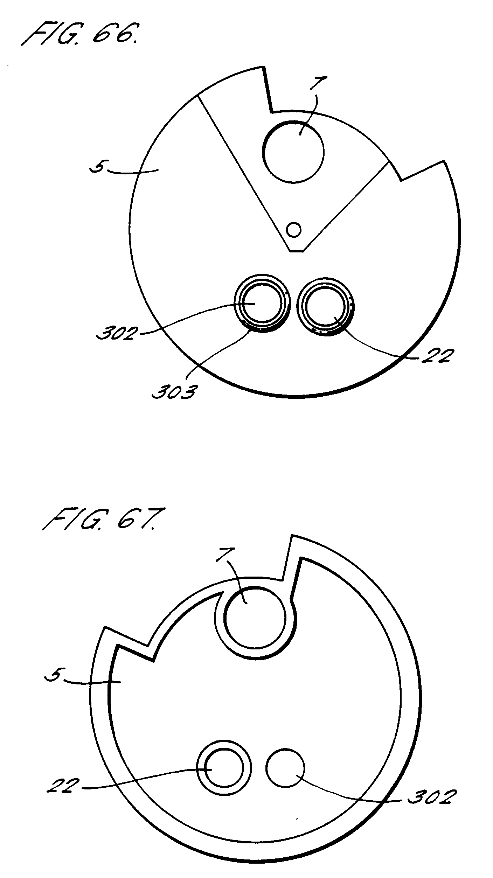

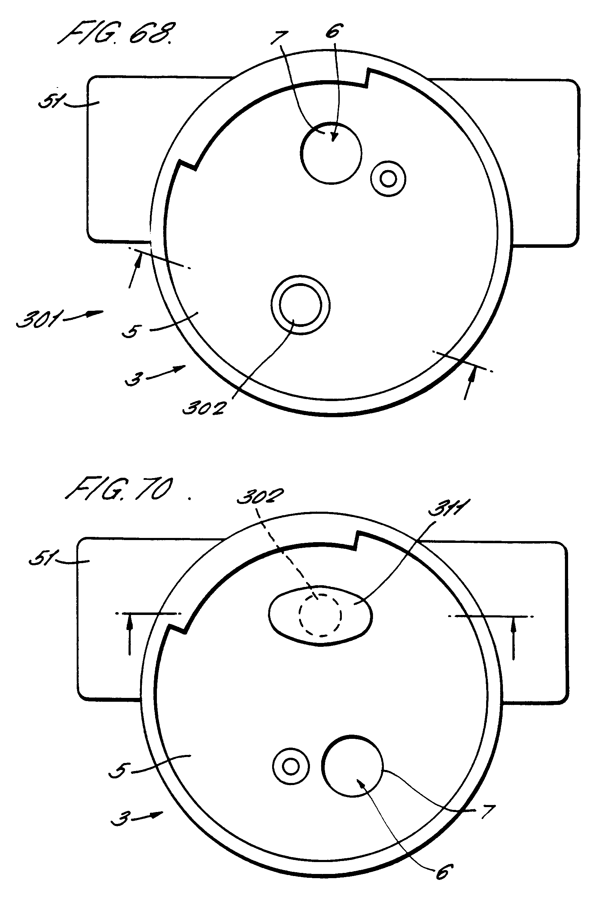

A resilient detent 63 is also provided adjacent the drive pin 30 and projects radially inwardly in the manner of a ratchet so as to cooperate with a ramped stop 64 formed on the rotatable portion 5 as shown in Figure 9. The detent 63 and stop 64 prevent reverse rotation of the actuating ring 46 relative to the rotatable portion 5 after completion of an initial stage of rotation between an initial position of the actuating ring (Figures 6, 15 & 16) and an actuated position (Figure 17).

Figure 15 shows the base 3 assembled with the rotatable portion 5 and the actuating ring 46 but with the suction cup not being shown.

The device 1 in Figure 15 is shown with the blade 18 in its initial position and with the arming device 29 in place so that relative movement between the actuating ring 46 and the rotatable portion 5 is prevented by the presence or the arming pin 32.

Also shown in Figure 15 is a plug 65 which closes a filling port 66 provided in the cover plate 52 to facilitate filling of the reservoir 11.

Figure 16 shows the device 1 after removal of the arming device 29, the pin 60 and detent 63 being thereby exposed in plan view.

Figure 17 shows the device 1 after subsequent rotation of the actuating ring 46 relative to the rotatable portion 5 from the initial position to the actuated position corresponding to the actuated position by the blade indicated schematically in Figure 2. In this actuated position, the blade 18 is seen to have traversed the access port 7 and will therefore have severed the suction cup 9 and suction blister 17 (not shown in Figure 17). In this actuated position the detent 63 can also be seen to have moved beyond the stop 64 and will subsequently prevent rotation of the actuating ring 46 relative to the rotatable portion 5.

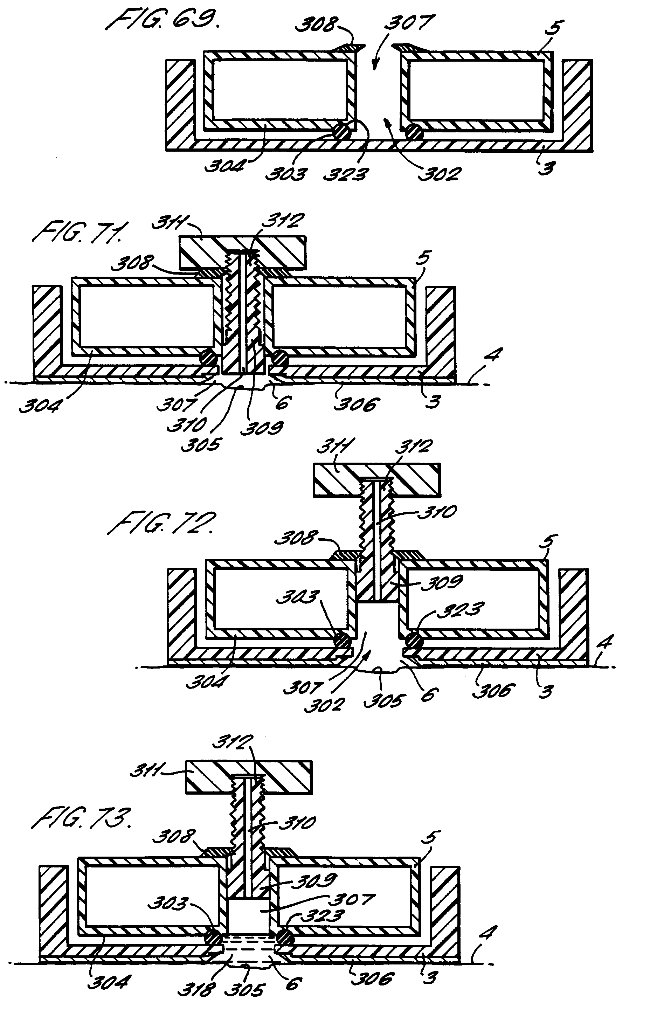

Figure 18 illustrates further clockwise rotation of the actuating ring 46 in unison with the rotatable portion 5 relative to the base 3 into the second position of the rotatable portion represented in Figure 3. In this second position the outlet port 22 of the reservoir 11 is brought into registration with the aperture 6 and the contents of the reservoir 11 enter the chamber 16. Absorption of the liquid drug 12 then occurs during a transdermal delivery phase of operation of the device.

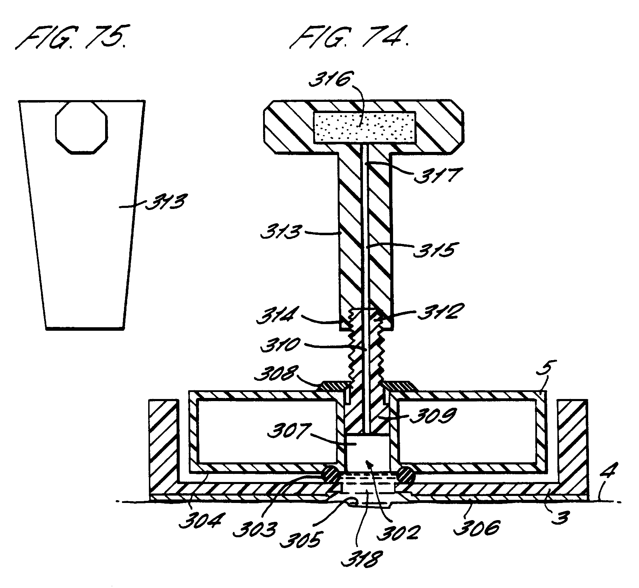

Figure 19 shows the final stage of operation in which reverse (anti-clockwise) rotation of the actuating ring 46 in unison with the rotatable portion 5 relative to the base 3 moves the rotatable portion into the third position represented in Figure 4. In this position the outlet port 22 of the reservoir 11 is closed and the ingress of air is prevented by the second O-ring seal 25 cooperating between the upper surface 23 of the base 3 and the bottom 54 of the rotatable portion 5.

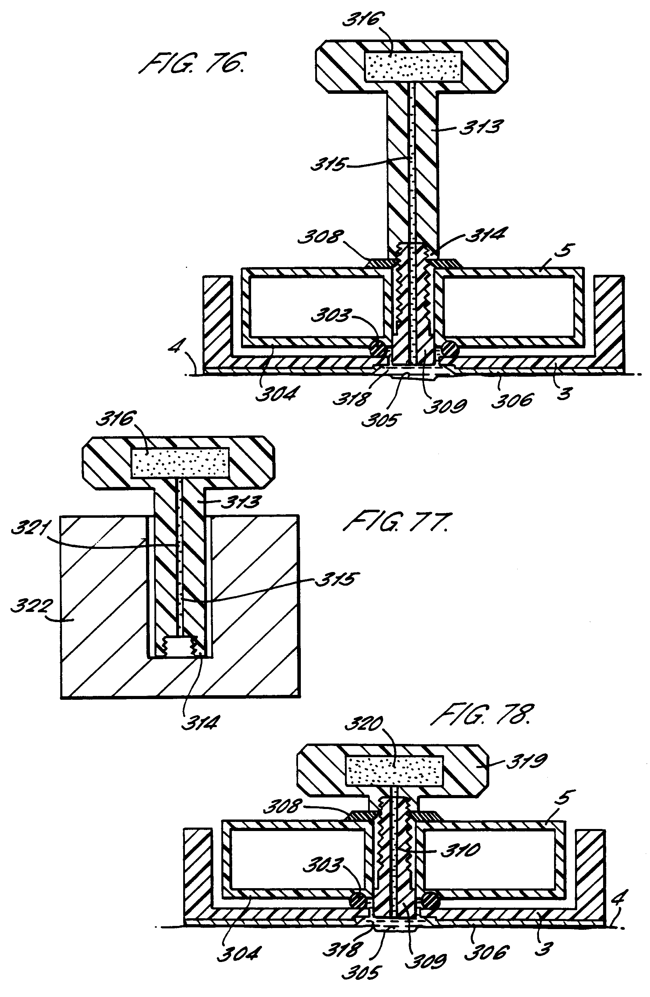

The device 1 of preceding figures relies upon manual actuation of an actuating ring 46 in order to achieve relative movement between the rotatable portion 5,70 and the stationary base 3. In order to assist the user in overcoming frictional forces between the relatively rotatable components, the device 1 may be modified as shown in Figure 21 to include an actuating mechanism 74 operable to achieve a mechanical advantage between movement of an actuating member 75 and the rotatable portion 5.

In the embodiment of Figure 21, the actuating member 75 consists of a rotatable key which is connected for rotation with a geared pinion 76 which engages a circumferential rack 77 formed peripherally of the rotatable portion 5.

The user may thereby more readily rotate the rotatable portion relative to the base 3 by winding the actuating member 75, the gear ratio in this example being 3:1.

Figures 27, 28 and 29 illustrate a preferred embodiment of the syringe 33 described above with reference to Figure 5. The syringe 33 comprises a piston 34 which is axially slidable within a cylinder 35 in order to increase the volume of a chamber 100 defined within the cylinder, thereby creating suction on demand.

The piston 34 is supported by an axially extending piston rod 101 of cruciform cross section which is integrally moulded with a transversely extending handle 102 and locking arms 103 and 104 which project from the handle so as to extend externally along the cylinder 35. The locking arms 103 and 104 are formed so as to be resiliently biased towards one another thereby tending to close around the cylinder 35.

The cylinder is provided with transversely projecting flanges 105,106 which are offset by 90 degrees about the axis or the cylinder with respect to both the radially extending handle 102 and the locking arms 103,104.

The locking arms 103 and 104 have respective free end portions 107 and 108 defining grooves 109 which indent the free end portions in an axial direction. The longitudinal extent of the locking arms 103 and 104 is less than that of the piston rod 101. In use, a user grips the handle 102 and pulls the handle away from flanges 105,106 so as to retract the piston 34 along the cylinder 35. When the displacement of the handle 102 is sufficient for the free end portions 107 and 108 to be pulled clear of the cylinder 35, the free end portions 107 and 108 spring radially inwardly towards one another. Suction created within the cylinder by retraction of the handle 102 will result in the piston 34 and handle 102 being biased against this direction of retraction so that by releasing the handle the piston will begin to travel in the return direction, this motion being arrested by engagement between the free end portions 107 and 108 and a lip 110 defining the end of the cylinder 35 such that the lip then extends into the slots 105 as shown in Figure 29.

Suction within the chamber 100 is thereby communicated to the suction cup of the device 1 by means of the suction tube 15 which is connected to a nose portion 111 of the syringe formed integrally with the cylinder 35.

Since the locking arms 103 and 104 are resiliently biased into the locking position, the syringe 33 is in effect self locking and operates simply by manually retracting the handle 102. Although it would be possible for the user to release the suction by first withdrawing the handle 102 and splaying apart the locking arms 103 and 104, this would require some co-ordinated manipulation or the handle 102, flanges 105,106 and the locking arms 103,104 so it is most unlikely that any accidental release of the suction in this manner could occur. The syringe 33 thereby provides a means operable to apply suction to the suction chamber 16 of the device 1 which is simple to use, self locking and inherently resistant to accidental de-activation.

As shown in Figure 66 the reservoir outlet port 22 is provided with an O-ring seal 24 and the sampling port 302 is provided with an O-ring seal 303. As shown in Figure 69, the O-ring seal 303 is operable between the upper surface 23 of the base 3 and the underneath surface 304 of the rotatable portion 5, the O-ring seal being captively retained relative to the rotatable portion by a locating groove so as to be rotatable in registration with the sampling port 302.

As shown in Figure 70, the rotatable portion 5 may be rotated into a position in which the sampling port 302 overlays the de-epithelialised erosion 305 in order to sample exudate generated at the skin erosion 305 from the exposed dermis. In this configuration, as shown in Figure 71, an air tight seal continues to be maintained between the base 3 and the skin 4 by means of an adhesive layer 306 which thereby provides an air tight seal. The O-ring seal 303 maintains this air tight seal between the base 3 and the rotatable portion 5 so that the sampling port 302 defines a cylindrical sampling chamber 307 into which exudate including plasma filtrate may fill.

A screw fitting 308 is mounted on the rotatable portion 5 at the upper entrance to the sampling chamber 307 thereby enabling a screw threaded sampling piston 309 to be inserted into the sampling chamber and advanced or retracted to any desired axial position thereby enabling the volume of the sampling chamber 307 to be variable. In Figure 71 the sampling piston 309 is shown in a fully advanced position in which the head of the piston is advanced into close proximity with the erosion 305 and volume of the sampling chamber 307 is thereby minimised. To initiate a sampling procedure, the sampling piston 309 is initially inserted into the sampling port 309 and advanced into the fully advanced position as shown in Figure 71. The sampling piston 309 includes an axially extending bore 310 allowing air trapped within the sampling chamber 307 to freely escape during the advancement of the sampling piston into the fully advanced position thereby avoiding any excess pressure in the sampling chamber which might otherwise have the undesirable effect of causing separation of the base 3 from the skin by disruption of the adhesive layer 306.

An occlusive cap 311 is then screw fitted to the outer end portion 312 of the sampling piston so as to close the bore 310 and constituting an openable closure. The piston 310 is then retracted by screw action to a fully retracted position as shown in Figure 72 thereby creating a partial vacuum in the sampling chamber 307, typically in the range 100 to 200 millimetres of mercury below atmospheric pressure.

This position is maintained for a period of approximately 15 minutes during which time exudate accumulates in the sampling chamber 307. The rate of production of exudate is enhanced by the partial vacuum created in the sampling chamber, the transdermal convective movement of liquid and entrained molecules from the plasma of the patient to the surface of the erosion being increased several fold. During this process, the intact semi-permeable capillary membrane of the dermis acts as a sieve to prevent significant contamination of the exudate liquid volume with red and white blood cells.

The occlusive cap 311 is then released from its screw fitting with the piston 309 and detached, thereby allowing air to enter the bore 310 and equalise pressure within the sampling chamber 307 with atmospheric pressure. A cuvette 313 as shown in Figures 74 and 75 is then fitted to the outer end portion 312 of the sampling piston 309 by means of a screw fitting 314 as shown in Figure 74, the cuvette defining a capillary slot 315 which when fitted to the piston lies in communication with the bore 310, the cuvette being formed of a transparent plastics material and having an absorbent pad 316 arranged to provide an air vent to an upper end 317 of the slot 315.

As shown in Figure 76 the sampling piston 309 may then be advanced into the sampling chamber 307 so as to displace the exudate 318 from the sampling chamber 307 into the cuvette 313 so as to fill the slot 315, excess exudate being absorbed by the pad 316.

The cuvette 313 may then be detached by reverse screw action from the sampling piston 309 and replaced by a plug 319 as shown in Figure 78 which is attachable by screw action to the sampling piston and includes a pad 320 of capillary active material for venting the sampling chamber to ambient air and collecting exudate continuing to eminante from the erosion 305 and collected via the bore 310.

The cuvette 313 containing a sample 321 of the exudate as shown in Figure 77 may then be inserted into an analysing apparatus 322 such as a photometer.

The cuvette also contains a reagent located on a sidewall of the slot 315 and selected to perform a chemical test on the sample. A range of reagents may be included in a single cuvette. The analysing apparatus may therefore be set up to respond to optically detectable changes in the reagent.

The alternative device 301 may also be used for the delivery of a drug to the erosion 305 by rotation of the rotatable portion 5 so as to bring the outlet port 22 of a drug reservoir within the rotatable portion into registration with aperture 6 formed in the base 3 in a manner corresponding to the arrangement described above with reference to Figure 3.

The alternative device may include other forms of openable closure, such as a valve mechanism.

The alternative device 301 may be provided with a skin heating element as described above with reference to Figure 22, the effect of heating being to enhance rates of sampling and delivery.

## Claims

1. Apparatus (1) for use in transdermal perfusion procedures comprising a housing (2), securing means (306) operable to secure a contact surface (50) of the housing in sealing contact with an area of skin (4) in use, an aperture (6) defined in the contact surface, a suction chamber (16) communicating with the aperture (6) and suction means (33) operable to apply suction to the suction chamber (16), characterised in that the aperture (6) communicates with an access port (7) defined by the housing, a suction cup (9) is located in the access port and has a lip portion (10) extending peripherally of the aperture and the suction cup (9) defines an outlet port (13), wherein the suction means (33) is operable to apply suction to the suction chamber (16) in the outlet port (13) and the apparatus further comprises cutting means (18) operable to sever from the lip portion a removable portion (20) of the suction cup (9) defining the outlet port (13).

2. Apparatus as claimed in claim 1 comprising an actuator (46) mounted on the housing and wherein the cutting means comprises a blade (18) which is movable to sever the removable portion in response to movement of the actuator relating to the housing.

3. Apparatus as claimed in any preceding claim wherein the suction means defines an expansion chamber (100) communicating with the outlet port via a tube (15), the suction means being operable to expand the volume of the expansion chamber from an initial volume to an expanded volume and further comprising locking means (103,104) operable to maintain the expansion chamber in its expanded volume.

4. Apparatus as claimed in claim 3 wherein the suction means comprises a syringe (33).

5. Apparatus as claimed in any of claims 3 and 4 comprising an indicator (38) responsive to displacement of air along the tube and operable to provide an indication of volumetric displacement of air from the suction chamber in response to the formation of a suction blister within the suction chamber.

6. Apparatus as claimed in claim 5 wherein the indicator comprises a slug (37) of liquid contained within the tube and indicating means (40) for indicating the extent of linear displacement of the slug of liquid through the tube.

7. Apparatus as claimed in claim 6 wherein the indicator comprises a clamp (39) securable at an adjustable position along the tube and wherein the indicating means is supported by the clamp.

8. Apparatus as claimed in any preceding claim wherein the suction means comprises a connector (14) which is releasably engagable with the outlet port whereby disengagement of the connector from the outlet port admits ambient air to the suction chamber.

9. Apparatus as claimed in claim 8 comprising an arming device (29) operable to prevent actuation of the cutting means until the connector has been disconnected from the outlet port.

10. Apparatus as claimed in claim 9 wherein the arming device comprises an arming pin (32) insertable into the housing to a location in which it prevents relative movement of the actuator and housing, the arming device further comprising a handle (31) connected to both the connector and the arming pin.

11. Apparatus as claimed in any preceding claim wherein the housing comprises a base (3) defining the contact surface and a rotatable portion (5) in which the access port is defined at an eccentric location relative to the rotation axis of the rotatable portion.

12. Apparatus as claimed in claim 11 wherein the rotatable portion comprises a reservoir (11) having an outlet (22) located eccentrically relative to the rotational axis such that, after the removable portion has been removed, the outlet is locatable by rotation of the rotatable portion in registration with the aperture defined in the base.

13. Apparatus as claimed in claim 12 comprising a continuous seal (24) extending peripherally of the outlet and operable between the rotatable portion and the base.

14. Apparatus as claimed in claim 13 comprising a second continuous seal (25) peripheral to a surface portion of the rotatable portion at a location which is eccentric relative to the rotational axis such that the surface portion is movable by rotation of the rotatable portion into registration with the aperture.

15. Apparatus as claimed in claim 14 wherein the continuous seal and the second continuous seal are integrally formed.

16. Apparatus as claimed in any of claims 11 to 15 comprising an actuating mechanism (75,76,77) operable to facilitate rotational movement of the rotatable portion relative to the base in response to movement of an actuating member (75) of the mechanism.

17. Apparatus as claimed in claim 16 wherein the mechanism comprises a geared pinion (76) mounted on the base for rotation by movement of the actuator and a circumferential rack (77) mounted on the rotatable portion and engaged by the pinion.

18. Apparatus as claimed in any of claims 11 to 17 wherein the rotatable portion defines a sampling port (302) at an eccentric location relative to the rotation axis of the rotatable portion, the apparatus further comprising a sampling piston (309) reciprocatable in the sampling port to vary the volume of a sampling chamber (307) defined therein whereby suction may be created in the sampling chamber, the sampling port being locatable, after the removable portion has been removed, by rotation of the rotatable portion in registration with the aperture defined in the base such that the aperture communicates with the sampling chamber.

19. Apparatus as claimed in claim 18 wherein the sampling piston defines a (310) bore to receive an outflow of fluid from the sampling chamber, the apparatus further comprising an openable closure (311) operable to close the bore to maintain suction in the sampling chamber.

20. Apparatus as claimed in claim 19 comprising a sampling device (313) connectable to the sampling piston and defining a sampling channel (315) communicating in use with the bore to receive a sample of fluid from the sampling chamber.

21. Apparatus as claimed in any of claims 18 to 20 wherein the sampling piston is reciprocatable in the sampling port by means of co-operable screw threaded formations or the sampling piston and the rotatable portion.

22. Apparatus as claimed in any preceding claim wherein the suction cup comprises a cylindrical portion (26) intermediate the lip and the outlet port and having a cylindrical axis substantially orthogonal to the contact surface and wherein the cutting means is operable to sever the cylindrical portion at a predetermined location spaced from the lip portion.

23. Apparatus as claimed in any preceding claim wherein the suction cup comprises an internal surface which is adhesively coated.

24. Apparatus as claimed in claim 1, wherein the suction means comprises a syringe (33) which comprises a cylinder (34) within which a piston (35) is movable to define an expansion chamber (100) of variable volume, the piston comprising an actuating handle (102) projecting from the cylinder and a locking arm (104) formed integrally therewith, the locking arm being resiliently biased for movement into a locking position in which a free end (108) of the locking arm engages a co-operating locking formation of the cylinder when the piston is in a retracted position to create and maintain suction in the expansion chamber.

25. Apparatus as claimed in claim 24 wherein the free end of the locking arm is indented to define a groove (109) engageable by an annular lip (110) of the cylinder constituting the co-operating locking formation.

26. Apparatus as claimed in any of claims 24 and 25 comprising a second locking arm (103) formed integrally with the handle, the respective locking arms extending longitudinally of the cylinder in diametrically opposed relationship thereto whereby the cylinder is resiliently gripped between the locking arms during movement of the piston into the retracted position.

## Patentansprüche

1. Vorrichtung (1) zur Verwendung bei transdermalen Perfusionsvorgängen, mit einem Gehäuse (2), Befestigungsmitteln (306), mit denen im Gebrauch eine Auflagefläche (50) des Gehäuses in abdichtender Anlage an einem Hautbereich (4) befestigbar ist, einer in der Auflagefläche definierten Öffnung (6), einer mit der Öffnung (6) kommunizierenden Saugkammer (16) und einer Saugeinrichtung (33), mit der Unterdruck an die Saugkammer (16) anlegbar ist,
dadurch **gekennzeichnet,** daß die Öffnung (6) mit einem vom Gehäuse definierten Zugangskanal (7) kommuniziert, daß ein Saugnapf (9) in dem Zugangskanal angeordnet ist und einen sich entlang des Umfangs der Öffnung erstreckenden Lippenabschnitt (10) aufweist und daß der Saugnapf (9) eine Auslaßöffnung (13) begrenzt, wobei die Saugeinrichtung (33) betreibbar ist, um einen Unterdruck an die Saugkammer (16) in der Auslaßöffnung (13) anzulegen, und daß die Vorrichtung ferner eine Schneideinrichtung (18) aufweist, durch deren Betätigung ein entfernbarer Abschnitt (20) des die Auslaßöffnung (13) definierenden Saugnapfes (9) von dem Lippenabschnitt abtrennbar ist.

2. Vorrichtung nach Anspruch 1,
mit einem in dem Gehäuse gelagerten Betätiger (46), wobei die Schneideinrichtung ein Messer (18) aufweist, welches durch Bewegung des Betätigers relativ zu dem Gehäuse bewegbar ist, um den entfernbaren Abschnitt abzutrennen.

3. Vorrichtung nach einem vorangehenden Anspruch,
bei der die Saugeinrichtung eine Expansionskammer (100) definiert, die über einen Schlauch (15) mit der Auslaßöffnung kommuniziert, wobei die Saugeinrichtung so betätigbar ist, daß das Volumen der Expansionskammer von einem Anfangsvolumen zu einem expandierten Volumen expandierbar ist, und ferner eine Verriegelungseinrichtung (103, 104) umfaßt, durch deren Betätigung die Expansionskammer in ihrem expandierten Volumen festlegbar ist.

4. Vorrichtung nach Anspruch 3,
bei der die Saugeinrichtung eine Spritze (33) umfaßt.

5. Vorrichtung nach einem der Ansprüche 3 und 4,
mit einem Indikator (38), der auf Bewegung von Luft längs des Schlauches anspricht und eine Anzeige für die volumetrische Entnahme von Luft aus der Saugkammer entsprechend der Bildung einer Saugblase in der Saugkammer liefert.

6. Vorrichtung nach Anspruch 5,
bei der der Indikator einen in dem Schlauch enthaltenen Pfropfen (37) von Flüssigkeit und Anzeigemittel (40) aufweist, die das Ausmaß der linearen Bewegung des Flüssigkeitspfropfens durch den Schlauch hindurch anzeigen.

7. Vorrichtung nach Anspruch 6,
bei der der Indikator eine Klammer (39) aufweist, die in einstellbarer Position längs des Schlauches befestigbar ist, wobei die Klammer das Anzeigemittel trägt.

8. Vorrichtung nach einem vorangehenden Anspruch,
bei der die Saugeinrichtung einen Verbinder (14) umfaßt, der lösbar mit der Auslaßöffnung koppelbar ist, wobei die Entkopplung des Verbinders von der Auslaßöffnung den Zutritt von Umgebungsluft zur Saugkammer bewirkt.

9. Vorrichtung nach Anspruch 8,
mit einer Sicherungsvorrichtung (39), die eine Betätigung der Schneideinrichtung verhindert, bis der Verbinder von der Auslaßöffnung entkoppelt ist.

10. Vorrichtung nach Anspruch 9,
bei der die Sicherungsvorrichtung einen Sicherungsstift (32) umfaßt, der in das Gehäuse in eine Stellung einsetzbar ist, an der er die Relativbewegung des Betätigers und des Gehäuses verhindert, und daß die Sicherungseinrichtung ferner einen Handgriff (31) aufweist, der sowohl mit dem Verbinder als auch mit dem Sicherungsstift verbunden ist.

11. Vorrichtung nach einem vorangehenden Anspruch,
bei der das Gehäuse eine die Auflagefläche definierende Basis und einen drehbaren Teil (5) aufweist, in dem der Zugangskanal in einer exzentrischen Anordnung relativ zur Drehachse des drehbaren Teils definiert ist.

12. Vorrichtung nach Anspruch 1,
bei der der drehbare Teil ein Reservoir (11) mit einem Auslaß (22) aufweist, der exzentrisch relativ zur Drehachse derart angeordnet ist, daß nach dem Entfernen des entfernbaren Teils der Auslaß durch Drehen des drehbaren Teils in Flucht mit der in der Basis definierten Öffnung einstellbar ist.

13. Vorrichtung nach Anspruch 12,
mit einer durchgehenden Dichtung (24), die sich um den Umfang des Auslasses erstreckt und zwischen dem drehbaren Teil und der Basis wirkt.

14. Vorrichtung nach Anspruch 13,
mit einer zweiten durchgehenden Dichtung (25), die sich um den Umfang eines Flächenbereichs des drehbaren Teils an einer Stelle erstreckt, die exzentrisch relativ zur Drehachse liegt, derart daß der Flächenbereich durch Drehen des drehbaren Teils in die Flucht mit der Öffnung drehbar ist.

15. Vorrichtung nach Anspruch 14,
bei der die durchgehende Dichtung und die zweite durchgehende Dichtung einstückig miteinander ausgebildet sind.

16. Vorrichtung nach einem der Ansprüche 14 bis 15,
mit einem Betätigungsmechanismus (75, 76, 77), durch dessen Betätigung die Drehbewegung des drehbaren Teils relativ zur Basis erleichtert wird in Abhängigkeit von der Bewegung eines Betätigungselementes (75) des Mechanismus.

17. Vorrichtung nach Anspruch 16,
bei der der Mechanismus ein Ritzel (76), das an der Basis gelagert und durch Bewegung des Betätigers drehbar ist, und einen Zahnkranz (77) aufweist, der an dem drehbaren Teil angeordnet ist und in den das Ritzel eingreift.

18. Vorrichtung nach einem der Ansprüche 11 bis 17,
bei der im drehbaren Teil ein Probenkanal (302) an einer zur Drehachse des drehbaren Teils exzentrischen Stelle definiert ist und die Vorrichtung ferner einen Probenentnahmekolben (309) aufweist, der in dem Probenkanal hin- und herbewegbar ist, um das Volumen einer in diesem begrenzten Probenkammer (307) zu verändern, wodurch ein Unterdruck in der Probenkammer erzeugbar ist, wobei der Probenkanal nach der Entfernung des entfernbaren Teils durch Drehen des drehbaren Teils in Flucht mit der in der Basis definierten Öffnung einstellbar ist, derart, daß die Öffnung mit der Probenkammer kommuniziert.

19. Vorrichtung nach Anspruch 18,
bei der der Probenentnahmekolben eine Bohrung (310) umgrenzt, die ausfließende Flüssigkeit aus der Probenkammer aufnehmen kann, wobei die Vorrichtung ferner einen zu öffnenden Verschluß (311) aufweist, mit dem die Bohrung verschließbar ist, um den Unterdruck in der Probenkammer aufrecht zu erhalten.

20. Vorrichtung nach Anspruch 19,
mit einer Probenentnahmevorrichtung (313), die mit dem Probenentnahmekolben verbindbar ist und einen Probenentnahmekanal (315) definiert, der bei Verwendung mit der Bohrung kommuniziert, um eine Flüssigkeitsprobe aus der Probenkammer aufzunehmen.

21. Vorrichtung nach einem der Ansprüche 18 bis 20,
bei der der Probenentnahmekolben in dem Probenkanal hin- und herbewegbar ist mittels zusammenwirkender Gewindeteile an dem Probenentnahmekolben und dem drehbaren Abschnitt.

22. Vorrichtung nach einem vorangehenden Anspruch,
bei der der Saugnapf einen zylindrischen Abschnitt (26) aufweist, der zwischen der Lippe und der Auslaßöffnung liegt und dessen Zylinderachse im wesentlichen rechtwinklig zur Auflagefläche verläuft, wobei die Schneideinrichtung betätigbar ist, um den zylindrischen Teil an einer vorgegebenen Stelle im Abstand von dem Lippenabschnitt durchzuschneiden.

23. Vorrichtung nach einem vorangehenden Anspruch,
bei der der Saugnapf eine Innenfläche aufweist, die mit einer Haftbeschichtung versehen ist.

24. Vorrichtung nach Anspruch 1,
bei der die Saugeinrichtung eine Spritze (33) aufweist mit einem Zylinder (34), in dem ein Kolben (35) bewegbar ist, um eine Expansionskammer (100) mit veränderbarem Volumen zu definieren, wobei der Kolben einen aus dem Zylinder herausragenden Betätigungshandgriff (102) und einen einstückig damit ausgebildeten Sperrarm (104) aufweist, wobei der Sperrarm unter Federbelastung steht für eine Bewegung in eine Sperrstellung, in der ein freies Ende (108) des Sperrarms in eine damit zusammenwirkende Sperraste des Zylinders eingreift, wenn sich der Kolben in einer zurückgezogenen Position befindet, um Unterdruck in der Expansionskammer zu erzeugen und aufrechtzuerhalten.

25. Vorrichtung nach Anspruch 24,
bei der das freie Ende des Sperrarms eine Einkerbung zur Bildung einer Nut (9) aufweist, in die eine Ringlippe (110) des Zylinders eingreifen kann, die die zusammenwirkende Sperrast bildet.

26. Vorrichtung nach einem der Ansprüche 24 und 26,
mit einem zweiten Sperrarm (103), der einstückig mit dem Handgriff ausgebildet ist, wobei die beiden Sperrarme sich jeweils in Längsrichtung des Zylinders einander diametral gegenüberliegend erstrecken, wodurch der Zylinder während der Bewegung des Kolbens in die zurückgezogene Stellung federnd zwischen den Sperrarmen ergriffen wird.

## Revendications

1. Appareil (1) destiné à être utilisé dans des opérations de perfusion transdermique comprenant un boitier (2), un dispositif (306) de fixation d'une surface (50) de contact du boîtier en contact étanche avec une région de la peau (4) pendant l'utilisation, un orifice (6) délimité dans la surface de contact, une chambre d'aspiration (16) communiquant avec l'orifice (6), et un dispositif (33) d'aspiration destiné à appliquer une aspiration à la chambre d'aspiration (16), caractérisé en ce que l'orifice (6) communique avec un canal d'accès (7) délimité par le boîtier, une cuvette (9) d'aspiration est placée dans le canal d'accès et a une partie (10) de lèvre disposée à la périphérie de l'orifice, et la cuvette d'aspiration (9) délimite un canal de sortie (13), dans lequel le dispositif d'aspiration (33) est destiné à appliquer une aspiration à la chambre d'aspiration (16) dans le canal de sortie (13), et l'appareil comporte en outre un dispositif de coupe (18) destiné à couper dans la partie de lèvre une partie amovible (20) de la cuvette d'aspiration (9) délimitant le canal de sortie (13).

2. Appareil selon la revendication 1, comprenant un organe de manoeuvre (46) monté sur le boîtier, et dans lequel le dispositif de coupe possède une lame (18) mobile afin qu'elle coupe la partie amovible lors du déplacement de l'organe de manoeuvre par rapport au boîtier.

3. Appareil selon l'une quelconque des revendications précédentes, dans lequel le dispositif d'aspiration délimite une chambre de dilatation (100) communiquant avec le canal de sortie par un tube (15), le dispositif d'aspiration étant destiné à dilater le volume de la chambre de dilatation d'un volume initial à un volume dilaté et comprenant en outre un dispositif de blocage (103, 104) destiné à maintenir la chambre de dilatation à son volume dilaté.

4. Appareil selon la revendication 3, dans lequel le dispositif d'aspiration est une seringue (33).

5. Appareil selon l'une des revendications 3 et 4, comprenant un indicateur (38) commandé par le déplacement de l'air le long du tube et destiné à donner une indication du déplacement volumétrique de l'air de la chambre d'aspiration lors de la formation d'une cloque d'aspiration dans la chambre d'aspiration.

6. Appareil selon la revendication 5, dans lequel l'indicateur comporte un tampon (37) de liquide contenu dans le tube et un dispositif indicateur (40) destiné à indiquer l'étendue du déplacement linéaire du tampon de liquide dans le tube.

7. Appareil selon la revendication 6, dans lequel l'indicateur comporte une pince (39) destinée à être fixée en position réglable le long du tube, et dans lequel le dispositif indicateur est supporté par la pince.

8. Appareil selon l'une quelconque des revendications précédentes, dans lequel le dispositif d'aspiration comporte un connecteur (14) qui peut coopérer de façon temporaire avec le canal de sortie si bien que la séparation du connecteur du canal de sortie provoque l'admission d'air ambiant dans la chambre d'aspiration.

9. Appareil selon la revendication 8, comprenant un dispositif d'armement (29) destiné à empêcher la manoeuvre du dispositif de coupe tant que le connecteur n'a pas été déconnecté du canal de sortie.

10. Appareil selon la revendication 9, dans lequel le dispositif d'armement comporte une broche d'armement (32) destinée à pénétrer dans le boîtier à un emplacement auquel elle empêche le déplacement relatif de l'organe de manoeuvre et du boîtier, le dispositif d'armement comprenant en outre une poignée (31) raccordée à la fois au connecteur et à la broche d'armement.

11. Appareil selon l'une quelconque des revendications précédentes, dans lequel le boîtier comporte une base (3) qui délimite la surface de contact et une partie rotative (5), et le canal d'accès est délimité à un emplacement excentrique par rapport à l'axe de rotation de la partie rotative.

12. Appareil selon la revendication 11, dans lequel la partie rotative est un réservoir (11) ayant une sortie (22) placée excentriquement par rapport à l'axe de rotation afin que, après l'enlèvement de la partie amovible, la sortie puisse être positionnée par rotation de la partie rotative en face de l'orifice délimité dans la base.

13. Appareil selon la revendication 12, comprenant un joint continu d'étanchéité (24) disposé à la périphérie de la sortie et utilisé entre la partie rotative et la base.

14. Appareil selon la revendication 13, comprenant un second joint continu d'étanchéité (25) placé à la périphérie d'une partie de surface de la partie rotative à un emplacement excentrique par rapport à l'axe de rotation afin que la partie de surface soit mobile par rotation de la partie rotative en position alignée sur l'orifice.

15. Appareil selon la revendication 14, dans lequel le joint continu d'étanchéité et le second joint continu d'étanchéité sont formés en une seule pièce.

16. Appareil selon l'une quelconque des revendications 11 à 15, comprenant un mécanisme de manoeuvre (75, 76, 77) destiné à faciliter le mouvement de rotation de la partie rotative par rapport à la base à la suite du déplacement de l'organe de manoeuvre (75) du mécanisme.

17. Appareil selon la revendication 16, dans lequel le mécanisme comprend un pignon (76) d'engrenage monté sur la base afin qu'il tourne lors du déplacement de l'organe de manoeuvre et une crémaillère circonférentielle (77) montée sur la partie rotative et coopérant avec le pignon.

18. Appareil selon l'une quelconque des revendications 11 à 17, dans lequel la partie rotative délimite un canal d'échantillonnage (302) à l'emplacement excentrique par rapport à l'axe de rotation de la partie rotative, l'appareil comprenant en outre un piston d'échantillonnage (309) qui peut se déplacer en translation dans le canal d'échantillonnage pour faire varier le volume d'une chambre d'échantillonnage (307) délimitée à l'intérieur de manière que l'aspiration puisse être créée dans la chambre d'échantillonnage, le canal d'échantillonnage pouvant être positionné, après enlèvement de la partie amovible, par rotation de la partie rotative en face de l'orifice délimité dans la base afin que l'orifice communique avec la chambre d'échantillonnage.

19. Appareil selon la revendication 18, dans lequel le piston d'échantillonnage délimite un trou (310) destiné à loger un courant de sortie de fluide de la chambre d'échantillonnage, l'appareil comprenant en outre un organe de fermeture (311) qui peut être ouvert et qui est destiné à fermer le trou pour permettre l'aspiration dans la chambre d'échantillonnage.

20. Appareil selon la revendication 19, comprenant un dispositif d'échantillonnage (313) qui peut être raccordé au piston d'échantillonnage et délimitant un canal d'échantillonnage (315) qui communique pendant l'utilisation avec le trou de la réception d'un échantillon de fluide de la chambre d'échantillonnage.

21. Appareil selon l'une quelconque des revendications 18 à 20, dans lequel le piston d'échantillonnage est mobile en translation dans le canal d'échantillonnage à l'aide d'organes conformés filetés coopérants du piston d'échantillonnage et de la partie rotative.

22. Appareil selon l'une quelconque des revendications précédentes, dans lequel la cuvette d'aspiration comprend une partie cylindrique (26) placée entre la lèvre et le canal de sortie et ayant un axe cylindrique pratiquement perpendiculaire à la surface de contact, et dans lequel le dispositif de coupe est destiné à découper la partie cylindrique à un emplacement prédéterminé à distance de la partie de lèvre.

23. Appareil selon l'une quelconque des revendications précédentes, dans lequel la cuvette d'aspiration est une surface interne revêtue d'un adhésif.

24. Appareil selon la revendication 1, dans lequel le dispositif d'aspiration est une seringue (33) qui comporte un cylindre (34) dans lequel est mobile un piston (35) pour la délimitation d'une chambre de dilatation (100) de volume variable, le piston comprenant une poignée de manoeuvre (102) qui dépasse du cylindre et un bras de blocage (104) formé afin qu'il en soit solidaire, le bras de blocage étant rappelé élastiquement afin qu'il se déplace en position de blocage dans laquelle une extrémité libre (108) du bras de blocage coopère avec un organe conformé coopérant de blocage du cylindre lorsque le piston est en position reculée afin qu'une aspiration soit créée et maintenue dans la chambre de dilatation.

25. Appareil selon la revendication 24, dans lequel l'extrémité libre du bras de blocage a une cavité destinée à délimiter une gorge (109) qui peut coopérer avec une lèvre annulaire (110) du cylindre constituant l'organe conformé coopérant de blocage.

26. Appareil selon l'une des revendications 24 et 25, comprenant un second bras de blocage (103) formé afin qu'il soit solidaire de la poignée, les bras respectifs de blocage étant disposés longitudinalement par rapport au cylindre en positions diamétralement opposées par rapport à celui-ci, si bien que le cylindre est saisi élastiquement entre les bras de blocage lors du déplacement du piston vers la position reculée.
